# EUROPEAN PATENT APPLICATION

(11) **EP 3 778 620 A1**
(43) Date of publication of application: **17.02.2021**
(21) Application number: 19777967.1
(22) Date of filing: 08.01.2019
(51) Int. Cl.: C07K 1/02, C07K 1/06

(54) **METHOD FOR PRODUCING LONG-CHAIN PEPTIDE**

(30) Priority: 29.03.2018 JP 2018065298
(71) Applicant: KANEKA CORPORATION, Osaka 530-8288 (JP)
(72) Inventor: MURATA, Takahiko, Takasago-shi, Hyogo 676-8688 (JP); YAMAMOTO, Shohei, Takasago-shi, Hyogo 676-8688 (JP); NISHIYAMA, Akira, Takasago-shi, Hyogo 676-8688 (JP)
(74) Representative: Hoffmann Eitle
(86) International application number: PCT/JP2019/000151
(87) International publication number: WO 2019/187469

(57) **Abstract**

The objective of the present invention is to provide a method for efficiently producing a long-chain peptide which method is suitable even for an industrial large scale production. The method for producing a long-chain peptide according to the present invention is characterized in comprising a step to deprotect an N-terminal site protected with BOC of a raw material peptide fragment with using a sulfonic acid compound, a step to selectively deprotect a C-terminal site protected with ONBn of a raw material peptide, and a step to condensate the obtained peptide fragment having the deprotected N-terminal site and the obtained peptide fragment having the deprotected C-terminal site in a liquid phase condition.

## Description

### TECHNICAL FIELD

The present invention relates to a method for efficiently producing a long-chain peptide.

### BACKGROUND ART

A long-chain peptide structure is a main structure of a peptide drug that has been attracting attention in recent years. Since the field of a peptide drug is expected to grow in the future, an efficient production method therefor has been desired in the market. A truly efficient production method therefor, however, has not been developed. For example, solid phase synthesis method is known as a typical method for producing a peptide. In solid phase synthesis method, a long-chain peptide is synthesized by using an amino acid immobilized on a solid phase as a starting raw material and adding each one of amino acids for elongation.

### Solid phase synthesis method

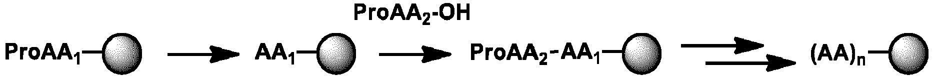

**AA = amino acid**
**Pro = Protecting Group**

Even a long-chain peptide composed of 100 or more amino acids can be synthesized by solid phase synthesis method. The method, however, is not necessarily efficient. For example, the method requires a large amount of reagents to complete each reaction, since all of the reactions are carried out on a solid phase. Thus, the method is not suitable for at least an industrial large scale production.

On the one hand, an amount of reagents can be significantly reduced and reactivity is higher in liquid phase synthesis method in comparison with solid phase synthesis method, since all of reactions are carried out in a liquid phase. When fragments are coupled in liquid phase synthesis method, however, it is needed to prepare a fragment of which protective group at the C-terminal site is selectively removed and a fragment of which protective group at the N-terminal site is selectively removed respectively. It is often difficult to select protective groups of each fragment. For example, a benzyl group is generally used for protecting a hydroxy group and a carboxy group, since a benzyl group can be easily removed by hydrogenation under a mild condition. When a C-terminal site hydroxy group, a side chain hydroxy group and a side chain carboxy group are protected with benzyl groups, however, only the C-terminal terminal site cannot be selectively deprotected by hydrogenation.

With respect to a protective group at a C-terminal site of a peptide fragment, a C-terminal site is protected with 4-nitrobenzyl ester group and deprotected in the presence of zinc powder in 90% acetic acid in the methods described in Non-patent documents 1 and 2. An amount of the zinc powder is not a catalytic amount. For example, an amount of the used zinc powder is 100 mg for deprotecting the C-terminal site of 273 mg of the peptide fragment. Thus, the method is not suitable for an industrial large scale production due to high environmental load, since a large amount of zinc powder is required for industrially producing a target peptide in a large scale and it is needed to treat a large amount of acetic acid after the reaction.

In addition, an N-terminal site is protected with Boc, i.e. t-butoxycarbonyl group, and deprotected by TFA, i.e. trifluoroacetic acid, in the methods described in Non-patent documents 1 and 2. This TFA must be completely removed by washing with n-hexane and drying in a vacuum with using KOH pellet after evaporation, since the TFA causes a side reaction during the next reaction to condensate a peptide fragment having deprotected N-terminal site and a peptide fragment having deprotected C-terminal site. The method is not considered to be efficient for producing a long-chain peptide, since the above procedure is needed whenever Boc group is removed.

### PRIOR ART DOCUMENT

### NON-PATENT DOCUMENT

Non-patent Document 1: TAKASHI ABIKO, et al., Chem. Pharm. Bull., 28(12), pp. 3542-3548 (1980)
Non-patent Document 2: TAKASHI ABIKO, et al., Chem. Pharm. Bull., 29(5), pp. 1390-1397 (1981)

### DISCLOSURE OF THE INVENTION

### PROBLEMS TO BE SOLVED BY THE INVENTION

As described above, liquid phase synthesis method is more suitable for industrially producing a long-chain peptide in large scale than solid phase synthesis method. On the one hand, it is often difficult to select a protective group and it is sometimes needed to completely remove a deprotecting reagent whenever an N-terminal site is deprotected in the case of liquid phase synthesis method.

Accordingly, the objective of the present invention is to provide a method for efficiently producing a long-chain peptide which method is suitable even for an industrial large scale production.

### MEANS FOR SOLVING THE PROBLEMS

The inventors of the present invention made extensive studies to solve the above problem. As a result, the inventors completed the present invention by finding that at least reactions from a deprotection of an N-terminal site to a condensation with a peptide fragment having a deprotected C-terminal site can be performed in the same system, i.e. in one-pot way, and it becomes easy to select a protective group for a reactive side chain functional group by protecting a C-terminal site of a peptide fragment with ONBn and deprotecting an N-terminal site protected with Boc of the peptide fragment with a sulfonic acid compound.

Hereinafter, the present invention is described.
[1] A method for producing a long-chain peptide, the method comprising:
   Step A to obtain a compound represented by the following formula (II) by selectively deprotecting an N-terminal site of a compound represented by the following formula (I) with using a sulfonic acid compound and then add a basic compound to neutralize a reaction mixture,

      Boc-(AA¹)ₘ-Pro¹ ··· (I)

      H- (AA¹)ₘ-Pro¹ ··· (II)

      in the formulae, Boc is a t-butoxycarbonyl group as a protective group at the N-terminal site, (AA¹)ₘ is a peptide chain wherein a reactive side chain functional group is protected, m is an integer of 2 or more and 50 or less, and Pro¹ is a protective group at a C-terminal site,
   Step B to obtain a compound represented by the following formula (IV) by selectively deprotecting a C-terminal site of a compound represented by the following formula (III),

      Boc-(AA²)ₙ-ONBn ··· (III)

      Boc-(AA²)ₙ-OH ··· (IV)

      in the formulae, (AA²)ₙ is a peptide chain wherein a reactive side chain functional group is protected, n is an integer of 2 or more and 50 or less, NBn is a nitrobenzyl group represented by the following formula (V) wherein p, q and r are independently 0 or 1, and p + q + r is 1, 2 or 3, 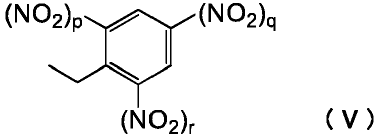
   and Step C to condensate the compound represented by the formula (II) and the compound represented by the formula (IV) by adding the compound represented by the formula (IV) to the reaction mixture of Step A to obtain a compound represented by the following formula (VI),

      Boc-(AA²)ₙ-(AA¹)ₘ-Pro¹ ··· (VI) in the formula, Boc, (AA²)ₙ, n, (AA¹)ₘ, m and Pro¹ have the same meanings as the above.
[2] The method according to the above [1], wherein the C-terminal site is selectively deprotected by using a combination of a metal and an acid or a dithionous acid compound.
[3] The method according to the above [1] or [2], wherein the sulfonic acid compound is 1 or more sulfonic acids selected from the group essentially consisting of methanesulfonic acid, trifluoromethanesulfonic acid and p-toluenesulfonic acid.
[4] The method according to any one of the above [1] to [3], wherein an organic amine compound is used as the basic compound.
[5] The method according to the above [4], wherein at least one of triethylamine and diisopropylamine is used as the organic amine compound.
[6] The method according to any one of the above [1] to [5], wherein Step A to Step C are repeated multiple times by obtaining the compound represented by the formula (VI) having ONBn as the Pro¹ in Step C and using the obtained compound represented by the formula (VI) as the compound represented by the formula (III) in Step B.
[7] The method according to any one of the above [1] to [6], wherein Step A to Step C are repeated multiple times by obtaining the compound represented by the formula (VI) having NH₂ or ONBn as the Pro¹ in Step C and using the obtained compound represented by the formula (VI) as the compound represented by the formula (I) in Step A.
[8] The method according to any one of the above [1] to [7], the method further comprising the step to deprotect the reactive side chain functional group of at least the compound represented by the formula (VI).
[9] The method according to any one of the above [1] to [8], wherein a solvent is used in Step C and an amide solvent is used as the solvent.
[10] The method according to the above [9], wherein the amide solvent is 1 or more amide solvents selected from the group essentially consisting of dimethylformamide, dimethylacetamide and dibutylformamide.
[11] The method according to any one of the above [1] to [10], wherein the long-chain peptide is exenatide.
[12] The method according to the above [8], wherein
   peptide fragments having the following amino acid sequences are used:
   F¹: Ala-Pro-Pro-Pro-Ser
   F²: Gly-Pro-Ser-Ser-Gly
   F³: Asn-Gly
   F⁴: Phe-Ile-Glu-Trp-Leu-Lys
   F⁵: Glu-Ala-Val-Arg-Leu
   F⁶: Ser-Lys-Gln-Met-Glu-Glu
   F⁷: Thr-Phe-Thr-Ser-Asp-Leu
   F⁸_{:} His-Gly-Glu-Gly,
   and the peptide fragment F^{s} wherein an N-terminal site is protected with Boc, a C-terminal site is protected with Pro¹, and s is any one of integers of 1 or more and 7 or less is condensated with the peptide fragment F^{s+1} wherein an N-terminal site is protected with Boc and C-terminal site is protected with ONBn by using the peptide fragment F^{s} as the compound represented by the formula (I) and using the peptide fragment F^{s+1} as the compound represented by the formula (III).
[13] The method according to the above [12], the method comprising
   a step to produce a peptide fragment F⁹ having the following amino acid sequence by using the peptide fragment F¹ wherein an N-terminal site is protected with Boc and a C-terminal site is protected with NH₂ as the compound represented by the formula (I) and using the peptide fragment F² wherein an N-terminal site is protected with Boc and a C-terminal site is protected with ONBn as the compound represented by the formula (III),
   F⁹: Gly-Pro-Ser-Ser-Gly-Ala-Pro-Pro-Pro-Ser
   a step to produce a peptide fragment F¹⁰ having the following amino acid sequence by using the peptide fragment F³ wherein an N-terminal site is protected with Boc and a C-terminal site is protected with ONBn as the compound represented by the formula (I) and using the peptide fragment F⁴ wherein an N-terminal site is protected with Boc and a C-terminal site is protected with ONBn as the compound represented by the formula (III),
   F¹⁰: Phe-Ile-Glu-Trp-Leu-Lys-Asn-Gly
   a step to produce a peptide fragment F¹¹ having the following amino acid sequence by using the peptide fragment F⁷ wherein an N-terminal site is protected with Boc and a C-terminal site is protected with ONBn as the compound represented by the formula (I) and using the peptide fragment F⁸ wherein an N-terminal site is protected with Boc and a C-terminal site is protected with ONBn as the compound represented by the formula (III),
   F¹¹: His-Gly-Glu-Gly-Thr-Phe-Thr-Ser-Asp-Leu
   a step to produce a peptide fragment F¹² having the following amino acid sequence by using the peptide fragment F¹⁰ wherein an N-terminal site is protected with Boc and a C-terminal site is protected with ONBn as the compound represented by the formula (I) and using the peptide fragment F⁵ wherein an N-terminal site is protected with Boc and a C-terminal site is protected with ONBn as the compound represented by the formula (III),
   F¹²: Glu-Ala-Val-Arg-Leu-Phe-Ile-Glu-Trp-Leu-Lys-Asn-Gly
   a step to produce a peptide fragment F¹³ having the following amino acid sequence by using the peptide fragment F⁹ wherein an N-terminal site is protected with Boc and a C-terminal site is protected with NH₂ as the compound represented by the formula (I) and using the peptide fragment F¹² wherein an N-terminal site is protected with Boc and a C-terminal site is protected with ONBn as the compound represented by the formula (III),
   F¹³: Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
   a step to produce a peptide fragment F¹⁴ having the following amino acid sequence by using the peptide fragment F¹³ wherein an N-terminal site is protected with Boc and a C-terminal site is protected with NH₂ as the compound represented by the formula (I) and using the peptide fragment F⁶ wherein an N-terminal site is protected with Boc and a C-terminal site is protected with ONBn as the compound represented by the formula (III),
   F¹⁴: Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
   a step to produce a peptide fragment F¹⁵ having the following amino acid sequence by using the peptide fragment F¹⁴ wherein an N-terminal site is protected with Boc and a C-terminal site is protected with NH₂ as the compound represented by the formula (I) and using the peptide fragment F¹¹ wherein an N-terminal site is protected with Boc and a C-terminal site is protected with ONBn as the compound represented by the formula (III),
   F¹⁵: His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
   and a step to deprotect an N-terminal site and a reactive side chain functional group of the peptide fragment F¹⁵.

### EFFECT OF THE INVENTION

Since all of the reactions of the method for producing a long-chain peptide according to the present invention are carried out in a liquid phase, a usage amount of reagents can be drastically reduced and a long-chain peptide can be efficiently produced in comparison with the case where a conventional solid phase synthesis method is applied. In addition, selecting an appropriate protective group for a C-terminal site has been a problem to be solved in a conventional liquid phase method; on the one hand, the protective group removable with a specific condition at a C-terminal site is selected in the present invention. As a result, it becomes easy to select a protective group of a reactive side chain functional group. Furthermore, a cumbersome procedure to completely remove a deprotecting reagent is not needed in the present invention, since even if there remains a deprotecting reagent, a peptide fragment having a deprotected N-terminal site and a peptide fragment having a deprotected C-terminal site can be condensated without difficulty by using the specific deprotecting reagent to remove a protective group at an N-terminal site. Accordingly, the present invention method is very useful industrially, since the present invention method is suitable for industrial large scale production of a long-chain peptide.

### MODE FOR CARRYING OUT THE INVENTION

Hereinafter, each step of the present invention method is described in detail. The phrase "compound represented by the formula (X)" is hereinafter abbreviated as "Compound (X)".

### Step A: Step to deprotect N-terminal site

In this step A, Compound (II) is obtained by selectively deprotecting an N-terminal site of Compound (I) with a sulfonic acid compound and then a reaction mixture is neutralized by adding a basic compound.

Boc-(AA¹)ₘ-Pro¹ ··· (I)

H- (AA¹)ₘ-Pro¹ ··· (II)

in the formulae, Boc is a t-butoxycarbonyl group as the protective group at the N-terminal site, (AA¹)ₘ is a peptide chain wherein a reactive side chain functional group is protected, m is an integer of 2 or more and 50 or less, and Pro¹ is a protective group at a C-terminal site.

The (AA¹)ₘ represents a peptide chain of which a reactive side chain functional group is protected and has the following structure unit. The AA¹ represents an amino acid residue, and a plurality of AA¹ may be the same as or different from each other. 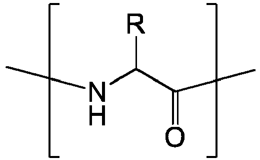 wherein R is a side chain of an amino acid.

It goes without saying that a side chain functional group which is not reactive, such as a side chain functional group of Gly, Ala, Val, Leu, Ile, Met, Asn, Gln, Pro and Phe, is not needed to be protected in (AA¹)ₘ. The term "reactive side chain functional group" means a highly reactive functional group in an amino acid side chain and is specifically exemplified by hydroxy group, thiol group, carboxy group, amino group, guanidino group and imidazole group. A methylthio group of Met and an amide group of Asn and Gln are not included in a reactive side chain functional group in this disclosure but may be protected to completely inhibit a side reaction. Specifically, a thioether group (-S-) in a side chain of Met may be subjected to sulfoxidation, and an example of a protective group of an amide group includes xanthyl, bis-2,4-dimethoxybenzyl and 4,4'-dimethoxybenzhydryl. A thiol group in a side chain of Cys may be oxidized. A thioether group in a side chain of Met and an amide group in a side chain of Asn and Gln are referred to as "quasi reactive side chain functional group" in some cases in this disclosure.

It is preferred that a protective group for a reactive side chain functional group is not deprotected in an acidic condition due to the sulfonic acid compound to deprotect an N-terminal site. A protective group for a hydroxy group in a side chain is exemplified by benzyl (Bzl), 4-methylbenzyl (4-MeBzl), 4-methoxybenzyl (4-MeOBzl) and 2-bromobenzyloxycarbonyl (Br-Z); a protective group for a thiol group in a side chain is exemplified by benzyl (Bzl), 4-methylbenzyl (4-MeBzl), 4-methoxybenzyl (4-MeOBzl) and t-butyl (tBu); a protective group for a carboxy group in a side chain is exemplified by benzyloxy (OBzl) and cyclohexyloxy (O-cHex); and a protective group for an amino group in a side chain is exemplified by formyl, 2,4,6-trimethylbenzensulfonyl (Mts), benzyloxycarbonyl (Z), nitro (NO₂), p-toluenesulfonyl (Tos), benzyloxymethyl (BOM), 2,4-dinitrophenyl (Dnp) and 2-chlorobenzyloxycarbonyl (Cl-Z); but the protective groups are not particularly restricted thereto.

Preferably, a protective group for a side chain of Ser and Thr is Bn group, a protective group for a side chain of Lys is 2-Cl-Z group or Z group, a protective group for a side chain of Trp is formyl group, a protective group for a side chain of Glu and Asp is Bn group, a protective group for a side chain of Arg is Z group or NO₂ group, a protective group for a side chain of His is BOM group, and a protective group for a side chain of Tyr is Bn group, Z group or 2-Br-Z group. A protective group removable by an acid, such as Boc, may be also used as a protective group for a side chain of an N-terminal amino acid.

The upper limit of "m" is not particularly restricted. When a peptide fragment is excessively large, it may possibly become difficult to progress the reaction. Thus, the "m" is preferably 100 or less, and more preferably 50 or less. The "m" is preferably 2 or more, more preferably 3 or more, and even more preferably 4 or more.

The Pro¹ represents a protective group for a C-terminal site. It is preferred that the protective group for a C-terminal site is not removed in an acidic condition due to the sulfonic acid compound to deprotect an N-terminal site among the protective groups described in Theodora W. Greene and Peter G. M. Wuts, "Protective Groups in Organic Chemistry Fourth Edition" published by WILEY-INTERSCIENCE, pp. 533-646. An example of such a protective group for the C-terminal site includes an ester protective group such as methyl ester, ethyl ester, benzyl ester, 4-nitrobenzyl ester, 2-nitrobenzyl ester, 2,4-dinitrobenzyl ester and 4-methoxybenzyl ester; and an amide protective group such as amide, dimethylamide, pyrrolidinylamide, piperidinylamide and o-nitroanilide.

When Compound (VI) obtained by Steps A to C is used as Compound (III) in Step B to newly repeat Steps A to C, a nitrobenzyl alcohol group (ONBn) selected from the group essentially consisting of 4-nitrobenzyl alcohol, 2-nitrobenzyl alcohol, 2,4-dinitrobenzyl alcohol, 4,6-dinitrobenzyl alcohol and 2,4,6-trinitrobenzyl alcohol is used as Pro¹.

Boc is used as a protective group for an N-terminal site of Compound (I), and the N-terminal site of Compound (I) is deprotected with using a sulfonic acid compound in this Step A. An N-terminal site protected with Boc is generally deprotected by using trifluoroacetic acid, but when trifluoroacetic acid is used, it is necessary to completely remove the trifluoroacetic acid in order to inhibit a side reaction. Such a procedure is not preferred in terms of a production efficiency and should be particularly avoided for an industrial large scale synthesis. On the one hand, when a reaction to remove Boc is carried out by using the sulfonic acid compound, an efficient production is possible, since the sulfonic acid compound is not needed to be removed and the reaction mixture can be subsequently used in the condensation step.

An example of the usable sulfonic acid compound includes methanesulfonic acid, trifluoromethanesulfonic acid and/or p-toluenesulfonic acid. A mixture of the compounds may be used. The sulfonic acid compound is particularly preferred from the standpoint that no significant by-product is generated even if the reaction mixture in which there remains the sulfonic acid compound is subjected to the coupling reaction in the next step without isolating the compound (II).

A usage amount of the sulfonic acid compound is not particularly restricted and may be appropriately adjusted, and may be adjusted to, for example, 0.1 times or more by mole and 400 times or less by mole to Compound (I). The amount is preferably 0.5 mol times or more by mole and 200 mol times or less by mole.

In this step A, a solvent may be used for the purpose that the liquid property of the reaction mixture is improved, the reaction is accelerated and a side reaction is inhibited. Such a solvent may be appropriately selected and is not particularly restricted, and is exemplified by water; an alcohol solvent such as methanol, ethanol, isopropanol, n-butanol and ethylene glycol; an aliphatic hydrocarbon solvent such as hexane and heptane; a halogenated hydrocarbon solvent such as dichloromethane, 1,2-dichloroethane, chloroform and chlorobenzene; an ether solvent such as tetrahydrofuran, 2-methyltetrahydrofuran, 1,4-dioxane, tert-butyl methyl ether and cyclopentyl methyl ether; an ester solvent such as ethyl acetate, isopropyl acetate and methyl propionate; a nitrile solvent such as acetonitrile, propionitrile and benzonitrile; a ketone solvent such as acetone, methyl ethyl ketone and acetophenone; an amide solvent such as dimethylformamide, dimethylacetamide, diethylformamide, dipropylformamide and dibutylformamide; and an aprotic polar solvent such as dimethylsulfoxide, N-methylpyrrolidone and 1,3-dimethylpropyleneurea. The solvent is preferably a halogenated hydrocarbon solvent, an ether solvent, an amide solvent and an aprotic polar solvent, more preferably a halogenated hydrocarbon solvent, an ether solvent and an amide solvent, and even more preferably 1 or more solvents selected from the group essentially consisting of dichloromethane, chlorobenzene, tetrahydrofuran, 2-methyltetrahydrofuran, dimethylformamide, dimethylacetamide and dibutylformamide. One of the solvents may be used alone or two or more of the solvents may be mixed to be used. When the solvents are mixed, a mixing ratio is not particularly restricted.

A usage amount of the solvent may be appropriately determined, and the usage amount to 1 g of Compound (I) may be 1 mL or more and 300 mL or less and preferably 5 mL or more and 100 mL or less.

An order to add each reagent is not particularly restricted, and for example, the sulfonic acid compound may be added to a mixture of Compound (I) and a solvent. A ratio of Compound (I) to the whole reaction mixture is preferably 0.2 W/V% or more and 50 W/V% or less and more preferably 1 W/V% or more and 20 W/V% or less.

A reaction temperature to deprotect the N-terminal site of Compound (I) is not particularly restricted and is preferably a boiling point of the used solvent or lower in terms of safety. The reaction temperature is preferably, for example, - 40°C or higher and 160°C or lower, more preferably -20°C or higher and 100°C or lower, and even more preferably 0°C or higher and 60°C or lower.

A reaction time to deprotect the N-terminal site of Compound (I) is also not particularly restricted, and may be determined by a preliminary experiment or the reaction may be continued until it is confirmed that Compound (I) is consumed by HPLC, thin-layer chromatography or the like. For example, the reaction time may be adjusted to 10 minutes or more and 24 hours or less.

In this step A, the N-terminal site of Compound (I) is deprotected and then the reaction mixture is neutralized by adding a basic compound.

The basic compound is not particularly restricted, may be an inorganic base or an organic base, and is preferably an organic base in terms of a solubility to the reaction mixture. An example of the basic compound includes an alkali metal hydroxide such as lithium hydroxide, sodium hydroxide, potassium hydroxide and cesium hydroxide; a carbonate salt of an alkali metal, such as lithium carbonate, sodium carbonate, potassium carbonate and cesium carbonate; a hydrogencarbonate salt of an alkali metal, such as lithium hydrogencarbonate, sodium hydrogencarbonate and potassium hydrogencarbonate; a metal alkoxide such as lithium methoxide, lithium ethoxide, lithium isopropoxide, lithium tert-butoxide, sodium methoxide, sodium ethoxide, sodium isopropoxide, sodium tert-butoxide, potassium methoxide, potassium ethoxide, potassium isopropoxide and potassium t-butoxide; a metal hydride such as sodium hydride, potassium hydride and calcium hydride; and an organic amine compound such as trimethylamine, triethylamine, tributylamine, diisopropylethylamine, N-methylpyrrolidine, N-methylmorpholine, 1,8-diazabicyclo[5,4,0]undec-7-ene, pyridine, quinoline and imidazole. One of the basic compounds may be used alone or two or more of the basic compounds may be used in combination. The basic compound is preferably an organic amine compound, and more preferably triethylamine and/or diisopropylethylamine.

A usage amount of the basic compound may be appropriately adjusted. For example, the usage amount may be adjusted such that a pH value of the reaction mixture after the basic compound is added becomes 6.5 or more. When the pH value is higher, the latter condensation reaction proceeds more successfully. Thus, the pH is preferably 6.5 or more and more preferably 7.0 or more. On the one hand, when the pH value is lower, an epimerization during the condensation reaction can be inhibited. Thus, the pH is preferably 10 or less and more preferably 8.0 or less.

An embodiment to add the basic compound is not particularly restricted. For example, after the deprotecting reaction at the N-terminal site of Compound (I) is completed, the basic compound may be directly added to the reaction mixture. Alternatively, the basic compound is dispersed or dissolved in a solvent, and the dispersion or solution may be added to the reaction mixture. As the solvent used in the above case, the solvent exemplified in the explanation of the deprotecting reaction at the N-terminal site of Compound (I) may be used. The same solvent as that used for the deprotecting reaction may be used or a different solvent may be used.

A temperature during the addition of the basic compound is not particularly restricted and may be appropriately determined. It is preferred that the basic compound is added after the reaction mixture is cooled, since a reaction heat may be sometimes generated due to the neutralization. For example, the basic compound is preferably added after the reaction mixture is cooled to -10°C or higher and 20°C or lower. It is also preferred that the reaction mixture is stirred during and after the addition of the basic compound.

### Step B: Step to deprotect C-terminal site

In this step B, Compound (IV) is obtained by selectively deprotecting the C-terminal site of Compound (III). This step B is preferably carried out separately from the above-described Step A.

Boc-(AA²)ₙ-ONBn ··· (III)

Boc-(AA²)ₙ-OH ··· (IV)

in the formulae, (AA²)ₙ is a peptide chain wherein a reactive side chain functional group is protected, n is an integer of 2 or more and 50 or less, NBn is a nitrobenzyl group represented by the following formula (V) wherein p, q and r are independently 0 or 1, and p + q + r is 1, 2 or 3.

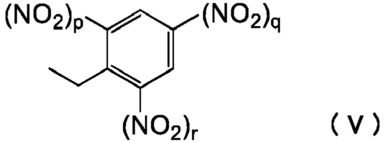

The (AA²)ₙ in the formula (III) and the formula (IV) is not necessarily the same as (AA¹)ₘ but the above-described explanation for (AA¹)ₘ in Step A can be directly applied to (AA²)ₙ. The "n" is preferably 100 or less, more preferably 50 or less, and preferably 2 or more, more preferably 3 or more, even more preferably 4 or more.

The NBn is specifically 1 or more nitrobenzyl groups selected from the group essentially consisting of 4-nitrobenzyl, 2-nitrobenzyl, 2,4-dinitrobenzyl, 4,6-dinitrobenzyl and 2,4,6-trinitrobenzyl. A benzyl-type protective group is used for protecting a hydroxy group and a carboxy group, such as a carboxy group at a C-terminal site, in a peptide, since a benzyl-type protective group can be easily removed by a hydrogenation reaction. On the one hand, when a general benzyl-type protective group is used for protecting a C-terminal site, the C-terminal site may not be selectively deprotected in some cases. In the present invention, a C-terminal site can be selectively deprotected by protecting the C-terminal site with ONBn.

Specifically, when there is not a reactive side chain functional group or a reactive side chain functional group is protected with only a protective group other than a benzyl-type protective group in (AA²)ₙ, NBn at the C-terminal site of Compound (III) can be easily removed by a general hydrogenation reaction. In addition, when there is a general benzyl-type protective group in a protective group to protect a reactive side chain functional group, only NBn at the C-terminal site can be selectively removed in a mild reductive condition. For more information, since a nitro group on a benzene ring in NBn can be easily reduced, only the nitro group of NBn can be reduced with maintaining a general benzyl-type protective group. When the nitro group in NBn is reduced, NBn is removed due to a subsequent electron transfer and only the C-terminal site can be deprotected.

A condition to reduce only the nitro group of NBn with maintaining a general benzyl-type protective group may be appropriately selected, and for example, a dithionous acid compound, which is a mild reducing agent, can be used. Specifically, a dithionous acid compound may be added to Compound (III) with a solvent, a dithionous acid compound may be added to a solution or a dispersion of Compound (III), or a solution or a dispersion of a dithionous acid compound may be added to a solution or a dispersion of Compound (III). An example of the dithionous acid compound includes sodium dithionite. Alternatively, NBn can be also selectively removed by using a reduction reaction with a combination of a metal and an acid. An example of such a metal includes Fe, Zn and Sn, and an example of such an acid includes ammonium chloride, acetic acid, hydrochloric acid and an acidic buffer.

A usage amount of the dithionous acid compound may be appropriately adjusted as long as the C-terminal site of Compound (III) can be selectively deprotected, and for example, 1 time or more by mole and 30 times or less by mole of the dithionous acid compound to Compound (III) can be used. The usage amount is preferably 20 times or less by mole, and preferably 3 times or more by mole, more preferably 5 times or more by mole. The term "time(s) by mole" means a molar number of an objective compound to 1 mole of a standard compound in this disclosure.

As a solvent usable in this Step B, the solvent exemplified in the explanation of the deprotecting reaction at the N terminal site of Compound (I) can be used. The solvent may be the same as or different from the solvent used in the deprotecting reaction. The solvent is preferably a mixed solvent of water and an amide solvent or a sulfoxide solvent, and more preferably a mixed solvent of water and 1 or more of organic solvents selected from the group essentially consisting of dimethylformamide (DMF), dimethylacetamide (DMA), dibutylformamide and dimethylsulfoxide (DMSO).

A reaction temperature of this Step B is not particularly restricted and is preferably a boiling point or lower of a used solvent in terms of safety. In addition, it is preferred to appropriately determine the temperature in consideration of the inhibition of impurity generation and the reaction rate. For example, the reaction temperature is preferably 20°C or higher and 150°C or lower, more preferably 30°C or higher and 100°C or lower, and even more preferably 50°C or higher and 90°C or lower.

Also, a reaction time of this Step B is not particularly restricted. The reaction time may be determined by a preliminary experiment, or the reaction may be continued until a consumption of Compound (III) is confirmed by HPLC, thin layer chromatography or the like. For example, the reaction time can be adjusted to 30 minutes or more and 24 hours or less.

A general posttreatment may be conducted after the reaction of this Step B. For example, the reaction mixture is cooled to room temperature, a poor solvent is added thereto to precipitate Compound (IV), and the precipitated Compound (IV) is collected by filtration, washed with a poor solvent and dried.

### Step C: Condensation step

In this Step C, Compound (VI) is obtained by adding Compound (IV) to the reaction mixture of the above-described Step A to condensate Compound (II) and Compound (IV). The reaction mixture of the above-described Step B and alternatively purified or roughly purified Compound (IV) may be added to the reaction mixture of the above-described Step A, and it is preferred that purified or roughly purified Compound (IV) is added to the reaction mixture of the above-described Step A. In other words, the above-described Step A and this Step C are performed within the same system, i.e. in a one-pot manner.

Boc-(AA² )ₙ-(AA')ₘ-Pro¹ ··· (VI)

in the formula, Boc, (AA² )ₙ, n, (AA¹)ₘ, m and Pro¹ have the same meanings as the above.

Usage amounts of Compound (II) and Compound (IV) in this Step C may be appropriately adjusted and for example, the amount to Compound (II) can be 0.4 times or more by mole and 5 times or less by mole, preferably 0.8 times or more by mole and 2 times or less by mole, and more preferably 0.9 times or more by mole and 1.5 times or less by mole.

A method for condensating Compound (II) and Compound (IV) in this Step C is not particularly restricted and for example, a carbodiimide compound or a dehydrating condensating agent is preferably used in the presence of an activating additive such as a hydroxyamine compound.

An example of the hydroxyamine compound includes 1-hydroxybenzotriazole (HOBt), 1-hydroxy-7-azabenzotriazole (HOAt), 3-hydroxy-4-oxo-3,4-dihydro-1,2,3-benzotriazine (HOOBt), N-hydroxysuccinimide (NHS), ethyl 2-oxime-cyanoglyoxylate (Oxyma), 1-hydroxy-6-chloro-benzotriazole (6-Cl-HOBt), N-hydroxyphthalimide and N-hydroxypiperidine, preferably 1-hydroxybenzotriazole (HOBt), 1-hydroxy-7-azabenzotriazole (HOAt) and 3-hydroxy-4-oxo-3,4-dihydro-1,2,3-benzotriazine (HOOBt), and more preferably 3-hydroxy-4-oxo-3,4-dihydro-1,2,3-benzotriazine (HOOBt).

A usage amount of the hydroxyamine compound may be appropriately adjusted and for example, the usage amount to Compound (III) may be 0.1 times or more by mole and 10 times or less by mole, and is preferably 0.5 times or more by mole and 5 times or less by mole.

An example of the carbodiimide compound includes dicyclohexylcarbodiimide (DCC) and 1-(3-dimethylaminopropyl)-3-ethylcarbodiimide hydrochloride (EDC), and preferably 1-(3-dimethylaminopropyl)-3-ethylcarbodiimide hydrochloride (EDC) in terms of easiness of posttreatment and accessibility.

An example of the dehydrating condensating agent includes 1H-benzotriazole-1-yloxy-tris-dimethylaminophosphonium hexafluorophosphate (BOP), 1H-benzotriazole-1-yloxy-tris-pyrrolidinophosphonium hexafluorophosphate (PyBOP), O-(benzotriazole-1-yl)-N,N,N' ,N'-tetramethyluronium hexafluorophosphate (HBTU), O-(7-azabenzotriazole-1-yl)-N,N,N',N'-tetramethyluronium hexafluorophosphate (HATU), O-(benzotriazole-1-yl)-N,N,N',N'-tetramethyluronium tetrafluoroborate (TBTU), O-[(ethoxycarbonyl)cyanomethylenamino]-N,N,N' ,N'-tetramethyluronium hexafluorophosphate (HOTU), (1-cyano-2-ethoxy-2-oxoethylideneaminooxy)dimethylamino-morpholino-carbenium hexafluorophosphate (COMU) and 4-(4,6-dimethoxy-1,3,5-triazine-2-yl)-4-methylmorpholinium chloride (DMT-MM), preferably O-(benzotriazole-1-yl)-N,N,N',N'-tetramethyluronium hexafluorophosphate (HBTU), O-(benzotriazole-1-yl)-N,N,N',N'-tetramethyluronium tetrafluoroborate (TBTU) and (1-cyano-2-ethoxy-2-oxoethylideneaminooxy)dimethylamino-morpholino-carbenium hexafluorophosphate (COMU), and more preferably O-(benzotriazole-1-yl)-N,N,N' ,N'-tetramethyluronium hexafluorophosphate (HBTU).

Usage amounts of the carbodiimide compound and the dehydrating condensating agent may be appropriately adjusted, are not particularly restricted, and for example, the usage amounts to Compound (III) may be 0.1 times or more by mole and 10 times or less by mole and preferably 0.5 times or more by mole and 5 times or less by mole.

A solvent may be used in this Step C. The same solvent exemplified in the explanation of the above-described Step A can be used as the solvent. When a solubility of the raw material in this Step C is low, the solvent preferably contains an amide solvent to accelerate the reaction by dissolving the raw material, and the solvent preferably contains 1 or more amide solvents selected from the group essentially consisting of dimethylformamide, dimethylacetamide and dibutylformamide. In order to use an amide solvent-containing solvent in this Step C, this Step C may be continuously started in the case where an amide solvent is used as at least a part of the solvent in the above-described Step A and Step B, or an amide solvent may be added in this Step C in the case where an amide solvent is not used in the above-described Step A and Step B. When an amide solvent is used as a part of the solvent in this Step C, a ratio of the amide solvent in the whole solvent in this Step C is preferably 2 v/v% or more, more preferably 30 v/v% or more, and even more preferably 70 v/v% or more. The above ratio means a ratio of the amide solvent before mixing to the total volume of each solvent before mixing.

A reaction temperature and a reaction time of this Step C are not particularly restricted and may be appropriately adjusted. For example, the reaction temperature may be adjusted to a boiling point or lower of the used solvent, is preferably adjusted to 50°C or lower in terms of an inhibition of a side reaction, and the reaction may be carried out under an ordinary temperature. The lower limit of the reaction temperature is not particularly restricted, and the reaction temperature is preferably -10°C or higher in terms of an effective progress of the reaction. The reaction time is not particularly restricted, and may be determined by a preliminary experiment, or the reaction may be continued until a consumption of Compound (II) or Compound (IV) is confirmed by HPLC, thin layer chromatography or the like. For example, the reaction time can be adjusted to 1 minute or more and 48 hours or less.

A general posttreatment may be conducted after the reaction of this Step C. For example, the reaction mixture is washed with water, saturated aqueous sodium chloride solution, saturated aqueous sodium hydrogencarbonate solution, sodium carbonate aqueous solution or the like, and then an organic phase is dried using anhydrous sodium sulfate or anhydrous magnesium sulfate and condensated. Compound (VI) may be further purified by column chromatography, crystallization procedure or the like.

The above Steps A to C may be repeated by using Compound (VI) obtained by this Step C as Compound (III) of the above Step B to extend the peptide chain. In such a case, ONBn is used as the protective group Pro¹ at the C-terminal site of Compound (VI) in common with Compound (III).

Alternatively, the above Steps A to C may be repeated by using Compound (VI) obtained by this Step C as Compound (I) of the above Step A to extend the peptide chain. In such a case, the protective group Pro¹ at the C-terminal site of Compound (VI) may be appropriately selected. For example, when exenatide described later is produced, exenatide can be efficiently produced by using NH₂ as Pro¹, since the C-terminal site of exenatide is -CONH₂. In addition, when Compound (VI) is used as a peptide chain in N-terminal side, Pro¹ may be ONBn.

### Step D: Step for deprotection

In this Step D, at least a reactive side chain functional group of Compound (VI) is deprotected to obtain a target peptide. It goes without saying that when AA² and AA¹ of Compound (VI) do not have a reactive side chain functional group or when the AA² or AA¹ has a quasi reactive side chain functional group but the quasi reactive side chain functional group is not protected, this Step D is not needed to be performed. If needed, at least one of the N-terminal site and the C-terminal site may be deprotected. A publically known condition can be applied as a condition to remove each protective group.

A general posttreatment may be conducted after the reaction of this Step D. For example, the reaction mixture is concentrated, and then a target peptide may be purified by column chromatography or the like.

A useful long chain peptide can be efficiently produced by the above-described present invention method. For example, the present invention method can be used for producing exenatide, which is a GLP-1 receptor agonist and which is widely used worldwide as a type 2 diabetes drug. Exenatide has an amino acid sequence of His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser (HGEGTFTSDLSKQMEEEAVRLFIEWLKNGGPSSGAPPPS), and the carboxy group at the C-terminal site thereof is amidated.

For example, when a long-chain peptide is produced by the present invention method, peptide fragments are designed by the cleavage of the long-chain peptide at an appropriate position where an epimerization is hardly caused, each peptide fragment is produced by the present invention method or a conventional method, and each peptide fragment may be condensated by the present invention method. In such a case, among neighboring peptide fragments, a peptide fragment of the C-terminal side is used as Compound (I) and a peptide fragment of the N-terminal side is used as Compound (III). When the peptide fragment has a reactive side chain functional group, a protective group which is not removed in the condition to selectively deprotect the N-terminal site and/or the C-terminal site, in other word, in the condition to remove Boc and/or NBn, is used as a protective group of the reactive side chain functional group.

For example, when exenatide is produced by the present invention method, the peptide fragments F¹ to F⁸ (Fragments 1 to 8) having the following amino acid sequences are designed toward the N-terminal site from the C-terminal site, and each peptide fragment may be condensated by the present invention method.
F¹: Ala-Pro-Pro-Pro-Ser
F²: Gly-Pro-Ser-Ser-Gly
F³: Asn-Gly
F⁴: Phe-Ile-Glu-Trp-Leu-Lys
F⁵: Glu-Ala-Val-Arg-Leu
F⁶: Ser-Lys-Gln-Met-Glu-Glu
F⁷: Thr-Phe-Thr-Ser-Asp-Leu
F⁸_{:} His-Gly-Glu-Gly

In the above peptide fragments, the C-terminal site of the peptide fragment F¹ corresponding to the C-terminal part of exenatide is preferably protected with NH₂, since the C-terminal site of exenatide is amidated and an amide group is relatively stable. Since the other peptide fragments F² to F⁷ are condensated with a peptide fragment in the C-terminal side in any one of the stages, ONBn is preferably used as the protective group of the C-terminal sites of the peptide fragments F² to F⁸.

More specifically, the peptide fragment F¹ used as Compound (I) and the peptide fragment F² used as Compound (III) are condensated to obtain the peptide fragment F⁹ having the following amino acid sequence,
F⁹: Gly-Pro-Ser-Ser-Gly-Ala-Pro-Pro-Pro-Ser
the peptide fragment F³ used as Compound (I) and the peptide fragment F⁴ used as Compound (III) are condensated to obtain the peptide fragment F¹⁰ having the following amino acid sequence,
F¹⁰: Phe-Ile-Glu-Trp-Leu-Lys-Asn-Gly
the peptide fragment F⁷ used as Compound (I) and the peptide fragment F⁸ used as Compound (III) are condensated to obtain the peptide fragment F¹¹ having the following amino acid sequence,
F¹¹: His-Gly-Glu-Gly-Thr-Phe-Thr-Ser-Asp-Leu
the peptide fragment F¹⁰ used as Compound (I) and the peptide fragment F⁵ used as Compound (III) are condensated to obtain the peptide fragment F¹² having the following amino acid sequence,
F¹²: Glu-Ala-Val-Arg-Leu-Phe-Ile-Glu-Trp-Leu-Lys-Asn-Gly
the peptide fragment F⁹ used as Compound (I) and the peptide fragment F¹² used as Compound (III) are condensated to obtain the peptide fragment F¹³ having the following amino acid sequence,
F¹³: Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
the peptide fragment F¹³ used as Compound (I) and the peptide fragment F used as Compound (III) are condensated to obtain the peptide fragment F¹⁴ having the following amino acid sequence,
F¹⁴: Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
the peptide fragment F¹⁴ used as Compound (I) and the peptide fragment F¹¹ used as Compound (III) are condensated to obtain the peptide fragment F¹⁵ having the following amino acid sequence,
F¹⁵: His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser.

Exenatide can be obtained by using NH₂ as the protective group of the C-terminal site of the above peptide fragment F¹, removing Boc as the protective group of the N-terminal site of the above peptide fragment F¹⁵ and deprotecting the reactive side chain functional group with an ordinary method.

The present application claims the benefit of the priority date of Japanese patent application No. 2018-65298 filed on March 29, 2018. All of the contents of the Japanese patent application No. 2018-65298 filed on March 29, 2018, are incorporated by reference herein.

### EXAMPLES

Hereinafter, the examples are described to demonstrate the present invention more specifically, but the present invention is in no way restricted by the examples, and the examples can be appropriately modified to be carried out within a range which adapts to the contents of this specification. Such a modified example is also included in the scope of the present invention.

### Example 1: Production of Fragment F⁹ from Fragment F¹ and Fragment F²

### (1) Production of Boc-Gly-Pro-Ser(Bzl)-Ser(Bzl)-Gly-OH

Na₂S₂O₄ (0.86 g, 5.0 mmol), DMA (5 mL) and water (0.5 mL) were added to Boc-Gly-Pro-Ser(Bzl)-Ser(Bzl)-Gly-O(4-NBn) (0.82 g, 1.0 mmol), and the mixture was stirred at 90°C for 2 hours. Ethyl acetate (8 mL) and water (8 mL) were added to the reaction mixture for extraction, and the aqueous layer and the oil layer were separated to obtain Boc-Gly-Pro-Ser(Bzl)-Ser(Bzl)-Gly-OH as Fragment F².

### (2) Production of Boc-Gly-Pro-Ser(Bzl)-Ser(Bzl)-Gly-Ala-Pro-Pro-Pro-Ser(Bzl)-NH₂

Dichloromethane (5 mL) and methanesulfonic acid (0.58 g, 6.0 mmol) were added to Boc-Ala-Pro-Pro-Pro-Ser(Bzl)-NH₂ (0.69 g, 1.1 mmol), and the mixture was stirred at room temperature for 1.5 hours. The reaction mixture was cooled in an ice bath, and triethylamine (0.62 g, 6.1 mmol) was added thereto. The mixture was stirred at room temperature for 10 minutes. Then, the reaction mixture was added to a dichloromethane solution of Boc-Gly-Pro-Ser(Bzl)-Ser(Bzl)-Gly-OH obtained in the above-described Example 1(1) (6 mL). Subsequently, HOBt·H₂O (0.23 g, 1.5 mmol) was added thereto and EDC hydrochloride (0.29 g, 1.5 mmol) was finally added thereto. The mixture was stirred at room temperature for 16 hours. Next, the reaction was stopped and Boc-Gly-Pro-Ser(Bzl)-Ser(Bzl)-Gly-Ala-Pro-Pro-Pro-Ser(Bzl)-NH₂ was obtained as Fragment F⁹ by extraction (0.93 mmol, purity: 95.0%).

### Example 2: Production of Fragment F¹⁰ from Fragment F³ and Fragment F⁴

### (1) Production of Boc-Phe-Ile-Glu(OBzl)-Trp(CHO)-Leu-Lys(Cl-Z)-OH

DMA (6 g), Na₂S₂O₄ (0.82 g, 4.7 mmol) and water (0.6 g) were added to Boc-Phe-Ile-Glu(OBzl)-Trp(CHO)-Leu-Lys(Cl-Z)-O(4-NBn) (1.0 g, 0.94 mmol), and the mixture was stirred at 90°C for 2 hours. Then, the mixture was cooled to 20°C, and 5% potassium hydrogensulfate aqueous solution (40 g) was added thereto. The precipitated solid was obtained by filtration and dried under reduced pressure to obtain 0.87 g of Boc-Phe-Ile-Glu(OBzl)-Trp(CHO)-Leu-Lys(Cl-Z)-OH as Fragment F⁴.

### (2) Production of Boc-Phe-Ile-Glu(OBzl)-Trp(CHO)-Leu-Lys(Cl-Z)-Asn-Gly-O(4-NBn)

Dichloromethane (160 mL) and methanesulfonic acid (5.4 g, 56 mmol) were added to Boc-Asn-Gly-O(4-NBn) (3.5 g, 8.3 mmol), and the mixture was stirred for 3 hours. The reaction mixture was cooled in an ice bath, and triethylamine (5.8 g, 57 mmol) was added thereto. The mixture was stirred for 5 minutes to deprotect the N-terminal site.

Boc-Phe-Ile-Glu(OBzl)-Trp(CHO)-Leu-Lys(Cl-Z)-OH (8.5 g, 7.0 mmol) obtained in the above-described Example 2(1) and HOOBt (2.0 g, 13 mmol) were added to a solution of H-Asn-Gly-0(4-NBn), and an aqueous solution (5.8 mL) of EDC hydrochloride (2.0 g, 10 mmol) was added thereto over 3 hours. The mixture was stirred at 20°C for 4 hours. Next, water (160 g) was added thereto and the precipitate of Boc-Phe-Ile-Glu(OBzl)-Trp(CHO)-Leu-Lys(Cl-Z)-Asn-Gly-O(4-NBn) was obtained by filtration as Fragment F¹⁰ (9.7 g, 6.3 mmol).

### Example 3: Production of Fragment F¹¹ from Fragment F⁷ and Fragment F⁸

### (1) Production of Boc-His(BOM)-Gly-Glu(OBzl)-Gly-OH

DMA (15 g), Na₂S₂O₄ (5.2 g, 30 mmol) and water (1.1 g) were added to Boc-His(BOM)-Gly-Glu(OBzl)-Gly-O(4-NBn) (5.0 g, 5.9 mmol), and the mixture was stirred at 100°C for 3 hours. The mixture was cooled to 10°C. Then, water (30 g) and dichloromethane (30 g) were added thereto for extraction, and an aqueous layer and an oil layer were separated to obtain Boc-His(BOM)-Gly-Glu(OBzl)-Gly-OH as Fragment F⁸.

### (2) Production of Boc-His(BOM)-Gly-Glu(OBzl)-Gly-Thr(Bzl)-Phe-Thr(Bzl)-Ser(Bzl)-Asp(OBzl)-Leu-O(4-NBn)

Dichloromethane (11 g) and methanesulfonic acid (2.4 g, 25 mmol) were added to Boc-Thr(Bzl)-Phe-Thr(Bzl)-Ser(Bzl)-Asp(OBzl)-Leu-O(4-NBn) (6.3 g, 4.9 mmol), and the mixture was stirred for 2 hours. The reaction mixture was cooled in an ice bath, and triethylamine (2.6 g, 25 mmol) was added thereto. The mixture was stirred for 15 minutes, and then a solution of Boc-His(BOM)-Gly-Glu(OBzl)-Gly-OH (42 g, 5.9 mmol) obtained in the above-described Example 3(1), HOBt·H₂O (1.1 g, 7.4 mmol) and EDC hydrochloride (1.4 g, 7.4 mmol) were added thereto in this order. After the mixture was stirred at 0°C for 18 hours, 5% sodium carbonate aqueous solution (60 g) was added thereto. The precipitated solid of Boc-His(BOM)-Gly-Glu(OBzl)-Gly-Thr(Bzl)-Phe-Thr(Bzl)-Ser(Bzl)-Asp(OBzl)-Leu-O(4-NBn) was obtained by filtration as Fragment F¹¹ (8.6 g, 4.6 mmol, purity: 93.5%).

### Example 4: Production of Fragment F¹² from Fragment F⁵ and Fragment F¹⁰

### (1) Production of Boc-Glu(OBzl)-Ala-Val-Arg(Z)₂-Leu-OH

DMA (60 mL), Na₂S₂O₄ (6.2 g, 36 mmol) and water (6 mL) were added to Boc-Glu(OBzl)-Ala-Val-Arg(Z)₂-Leu-O(4-NBn) (8.5 g, 7.2 mmol), and the mixture was stirred at 90°C for 4 hours. Then, water (60 mL) was added thereto, and the mixture was cooled to room temperature. Ethyl acetate (60 mL) was added thereto, and the mixture was stirred at room temperature for 2 hours. The precipitated solid was obtained by filtration and washed with water and ethyl acetate to obtain Boc-Glu(OBzl)-Ala-Val-Arg(Z)₂-Leu-OH as Fragment F⁵ (7.5 g, 7.2 mmol, Purity: 96.5%).

### (2) Production of Boc-Glu(OBzl)-Ala-Val-Arg(Z)₂-Leu-Phe-Ile-Glu(OBzl)-Trp(CHO)-Leu-Lys(Cl-Z)-Asn-Gly-O(4-NBn)

Dichloromethane (105 mL) and methanesulfonic acid (3.0 g, 31 mmol) were added to Boc-Phe-Ile-Glu(OBzl)-Trp(CHO)-Leu-Lys(Cl-Z)-Asn-Gly-O(4-NBn) (9.5 g, 6.2 mmol) produced in the above-described Example 2, and the mixture was stirred for 1 hour. The reaction mixture was cooled in an ice bath, and triethylamine (2.8 g, 28 mmol) was added thereto. The mixture was stirred for 5 minutes. DMA (146 g) was further added thereto, and the mixture was warmed up to 20°C.

Boc-Glu(OBzl)-Ala-Val-Arg(Z)₂-Leu-OH (6.5 g, 6.2 mmol) produced in the above-described Example 4(1) and HOOBt (1.5 g, 9.3 mmol) were added to the above reaction mixture, and DMA solution (311 mL) of EDC hydrochloride (1.8 g, 9.3 mmol) was finally added thereto over 5 hours. The mixture was stirred at 20°C for 13 hours and then at 30°C for 17 hours. Further, HOOBt (0.25 g, 1.6 mmol), EDC hydrochloride (0.30 g, 1.6 mmol), Boc-Glu(OBzl)-Ala-Val-Arg(Z)₂-Leu-OH (0.97 g, 0.94 mmol) and triethylamine (0.22 g, 2.2 mmol) were added thereto, and the mixture was stirred for 15 hours. Then, water (570 mL) was added thereto. After a part of the mixture was concentrated at 40°C under reduced pressure. Water (285 mL) was added again. The mixture was cooled to 0°C, and the precipitate of Boc-Glu(OBzl)-Ala-Val-Arg(Z)₂-Leu-Phe-Ile-Glu(OBzl)-Trp(CHO)-Leu-Lys(Cl-Z)-Asn-Gly-O(4-NBn) was obtained by filtration as Fragment F¹² (15.7 g) .

### Example 5: Production of Fragment F¹³ from Fragment F⁹ and Fragment F¹²

### (1) Production of Boc-Glu(OBzl)-Ala-Val-Arg(Z)₂-Leu-Phe-Ile-Glu(OBzl)-Trp(CHO)-Leu-Lys(Cl-Z)-Asn-Gly-OH

DMA (90 g) was added to Boc-Glu(OBzl)-Ala-Val-Arg(Z)₂-Leu-Phe-Ile-Glu(OBzl)-Trp(CHO)-Leu-Lys(Cl-Z)-Asn-Gly-O(4-NBn) (15 g, 6.1 mmol) produced in the above-described Example 4, and the mixture was warmed up to 55°C. Then, water (9.0 g) and Na₂S₂O₄ (5.3 g, 30 mmol) were added thereto, and the mixture was heated up to 90°C. After the mixture was stirred at 90°C for 2 hours, water (90 g) was added and the precipitate of Boc-Glu(OBzl)-Ala-Val-Arg(Z)₂-Leu-Phe-Ile-Glu(OBzl)-Trp(CHO)-Leu-Lys(Cl-Z)-Asn-Gly-OH was obtained by filtration as Fragment F¹² (13.4 g, 5.8 mmol).

### (2) Production of Boc-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂

Methanesulfonic acid (3.7 g, 39 mmol) was added to the dichloromethane solution (96 g) of Boc-Gly-Pro-Ser(Bzl)-Ser(Bzl)-Gly-Ala-Pro-Pro-Pro-Ser(Bzl)-NH₂ (7.1 g, 5.8 mmol) obtained in the above-described Example 1, and the mixture was stirred for 3 hours. The reaction mixture was cooled in an ice bath. Triethylamine (4.2 g, 41 mmol) was added thereto, and the mixture was stirred for 5 minutes. After DMA (106 g) was further added thereto, dichloromethane was distilled away under reduced pressure.

Then, Boc-Glu(OBzl)-Ala-Val-Arg(Z)₂-Leu-Phe-Ile-Glu(OBzl)-Trp(CHO)-Leu-Lys(Cl-Z)-Asn-Gly-OH (11.2 g, 4.8 mmol) obtained in the above-described Example 5(1), DMA (106 g) and HOOBt (1.6 g, 9.7 mmol) were added thereto, and EDC hydrochloride (1.9 g, 9.7 mmol) was finally added. The mixture was stirred for 2 hours. Further, EDC hydrochloride (0.46 g, 2.4 mmol) and triethylamine (0.24 g, 2.4 mmol) were added 3 times every 1 hour, and the mixture was stirred for 1 hour. Next, triethylamine (0.49 g, 4.8 mmol) was added, and the mixture was stirred for 13 hours. Water (106 g) was added, and the precipitate of Boc-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂ was obtained by filtration as Fragment F¹³ (13.3 g, 3.9 mmol).

### Example 6: Production of Fragment F¹⁴ from Fragment F¹³ and Fragment F⁶

### (1) Production of Boc-Ser(Bzl)-Lys(Cl-Z)-Gln-Met(O)-Glu(OBzl)-Glu(OBzl)-OH

DMA (6.0 g), water (0.6 g) and Na₂S₂O₄ (1.2 g, 6.9 mmol) were added to Boc-Ser(Bzl)-Lys(Cl-Z)-Gln-Met(O)-Glu(OBzl)-Glu(OBzl)-O(4-NBn) (1.0 g, 0.69 mmol), and the mixture was stirred at 90°C for 4 hours. Water (60 g) was subsequently added, and the precipitated solid of Boc-Ser(Bzl)-Lys(Cl-Z)-Gln-Met(O)-Glu(OBzl)-Glu(OBzl)-OH was obtained by filtration as Fragment F⁶ (0.89 g, 0.68 mmol, Purity: 94.6%).

### (2) Production of Boc-Ser(Bzl)-Lys(Cl-Z)-Gln-Met(O)-Glu(OBzl)-Glu(OBzl)-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂

Dichloromethane (104 g) was added to Boc-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂ (13 g, 3.8 mmol) obtained in the above-described Example 5. Methanesulfonic acid (7.3 g, 76 mmol) was subsequently added, and the mixture was stirred for 2 hours. The reaction mixture was cooled in an ice bath. Triethylamine (8.3 g, 82 mmol) was added thereto, and the mixture was stirred for 5 minutes. DMA (169 g), Boc-Ser(Bzl)-Lys(Cl-Z)-Gln-Met(O)-Glu(OBzl)-Glu(OBzl)-OH (3.9 g, 3.0 mmol) obtained in the above-described Example 6(1) and HOOBt (1.2 g, 7.6 mmol) were added thereto. Subsequently, EDC hydrochloride (0.36 g, 1.9 mmol) was added 4 times every 30 minutes. After the mixture was stirred for 1 hour, Boc-Ser(Bzl)-Lys(Cl-Z)-Gln-Met(O)-Glu(OBzl)-Glu(OBzl)-OH (2.0 g, 1.5 mmol) was added. The mixture was stirred for 2 hours. Triethylamine (0.19 g, 1.9 mmol) and EDC hydrochloride (0.36 g, 1.9 mmol) were added again, and the mixture was stirred for 13 hours. After water (273 g) was added and the mixture was concentrated under reduced pressure, water (104 g) was added again, and the precipitated solid of Boc-Ser(Bzl)-Lys(Cl-Z)-Gln-Met(O)-Glu(OBzl)-Glu(OBzl)-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂ was obtained by filtration as Fragment F¹⁴ (16.1 g).

### Example 7: Production of Fragment F¹⁵ from Fragment F¹⁴ and Fragment F¹¹

### (1) Production of Boc-His(BOM)-Gly-Glu(OBzl)-Gly-Thr(Bzl)-Phe-Thr(Bzl)-Ser(Bzl)-Asp(OBzl)-Leu-OH

DMSO (81 g) was added to Boc-His(BOM)-Gly-Glu(OBzl)-Gly-Thr(Bzl)-Phe-Thr(Bzl)-Ser(Bzl)-Asp(OBzl)-Leu-O(4-NBn) (11 mmol) obtained in the above-described Example 3, and the mixture was warmed to 45°C. Na₂S₂O₄ (5.6 g, 32 mmol) was added thereto, and the mixture was stirred at 65°C for 7 hours. Then, Na₂S₂O₄ (1.9 g, 11 mmol) was added again, and the mixture was stirred for 3 hours. Water (0.58 g) and Na₂S₂O₄ (2.8 g, 16 mmol) were added. The mixture was stirred for 1 hour and then at 70°C for 3 hours. After the mixture was cooled to 20°C, water (302 g) was added and the precipitate of Boc-His(BOM)-Gly-Glu(OBzl)-Gly-Thr(Bzl)-Phe-Thr(Bzl)-Ser(Bzl)-Asp(OBzl)-Leu-OH was obtained by filtration as Fragment F¹¹ (20 g).

### (2) Production of Boc-His(BOM)-Gly-Glu(OBzl)-Gly-Thr(Bzl)-Phe-Thr(Bzl)-Ser(Bzl)-Asp(OBzl)-Leu-Ser(Bzl)-Lys(Cl-Z)-Gln-Met (O)-Glu(OBzl) -Glu (OBzl) -Glu (OBzl) -Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂

Dichloromethane (177 g) was added to Boc-Ser(Bzl)-Lys(Cl-Z)-Gln-Met(O)-Glu(OBzl)-Glu(OBzl)-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂ (15 g, 3.2 mmol) obtained in the above-described Example 6. Methanesulfonic acid (9.3 g, 96 mmol) was subsequently added, and the mixture was stirred for 2 hours. The reaction mixture was cooled in an ice bath. Triethylamine (10 g, 103 mmol) was added, and the mixture was stirred for 5 minutes. DMA (177 g) was added, and Boc-His(BOM)-Gly-Glu(OBzl)-Gly-Thr(Bzl)-Phe-Thr(Bzl)-Ser(Bzl)-Asp(OBzl)-Leu-OH (6.1 g, 3.5 mmol) obtained in the above-described Example 7(1) and HOOBt (1.1 g, 6.4 mmol) were further added. EDC hydrochloride (1.2 g, 6.4 mmol) was subsequently added, and the mixture was stirred for 4 hours. Boc-His(BOM)-Gly-Glu(OBzl)-Gly-Thr(Bzl)-Phe-Thr(Bzl)-Ser(Bzl)-Asp(OBzl)-Leu-OH (0.56 g, 0.32 mmol) was further added, and the mixture was stirred for 3 hours. The reaction mixture was subsequently concentrated under reduced pressure. After water (354 g) was added to the residue and the mixture was cooled to 10°C, the precipitated solid of Boc-His(BOM)-Gly-Glu(OBzl)-Gly-Thr(Bzl)-Phe-Thr(Bzl)-Ser(Bzl)-Asp(OBzl)-Leu-Ser(Bzl)-Lys(Cl-Z)-Gln-Met(O)-Glu(OBzl)-Glu(OBzl)-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂ was obtained by filtration as Fragment F¹⁵ (19.3 g).

### Example 8: Production of exenatide

Dichloromethane (15 g) was added to Boc-His(BOM)-Gly-Glu(OBzl)-Gly-Thr(Bzl)-Phe-Thr(Bzl)-Ser(Bzl)-Asp(OBzl)-Leu-Ser(Bzl)-Lys(Cl-Z)-Gln-Met(O)-Glu(OBzl)-Glu(OBzl)-Glu(OBzl)-Ala-Val-Arg(Z)₂-Leu-Phe-Ile-Glu(OBzl)-Trp(CHO)-Leu-Lys(Cl-Z)-Asn-Gly-Gly-Pro-Ser(Bzl)-Ser(Bzl)-Gly-Ala-Pro-Pro-Pro-Ser(Bzl)-NH₂ (1.0 g, 0.2 mmol) obtained in the above-described Example 7. Methanesulfonic acid (1.2 g, 13 mmol) was subsequently added, and the mixture was stirred for 1 hour. The reaction mixture was cooled in an ice bath, DMF (10 g) was added thereto, and the mixture was concentrated under reduced pressure. Then, after hydrogenation reaction was carried out using Pd/C under hydrogen atmosphere, piperidine was added to obtain exenatide.

## Claims

1. A method for producing a long-chain peptide, the method comprising:
Step A to obtain a compound represented by the following formula (II) by selectively deprotecting an N-terminal site of a compound represented by the following formula (I) with using a sulfonic acid compound and then add a basic compound to neutralize a reaction mixture,
BOc-(AA¹)ₘ-Pro¹ ··· (I)
H- (AA¹)ₘ-Pro¹ ··· (II)
in the formulae, Boc is a t-butoxycarbonyl group as a protective group at the N-terminal site, (AA¹)ₘ is a peptide chain wherein a reactive side chain functional group is protected, m is an integer of 2 or more and 50 or less, and Pro¹ is a protective group at a C-terminal site,
Step B to obtain a compound represented by the following formula (IV) by selectively deprotecting a C-terminal site of a compound represented by the following formula (III),
Boc-(AA²)ₙ-ONBn ··· (III)
Boc-(AA²)ₙ-OH ··· (IV)
in the formulae, (AA²)ₙ is a peptide chain wherein a reactive side chain functional group is protected, n is an integer of 2 or more and 50 or less, NBn is a nitrobenzyl group represented by the following formula (V) wherein p, q and r are independently 0 or 1, and p + q + r is 1, 2 or 3, 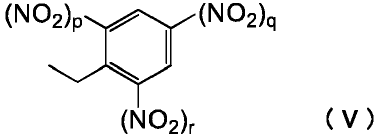 and Step C to condensate the compound represented by the formula (II) and the compound represented by the formula (IV) by adding the compound represented by the formula (IV) to the reaction mixture of Step A to obtain a compound represented by the following formula (VI),
Boc- (AA²)ₙ- (AA¹)ₘ-Pro¹ ··· (VI)
in the formula, Boc, (AA²)ₙ, n, (AA¹)ₘ, m and Pro¹ have the same meanings as the above.

2. The method according to claim 1, wherein the C-terminal site is selectively deprotected by using a combination of a metal and an acid or a dithionous acid compound.

3. The method according to claim 1 or 2, wherein the sulfonic acid compound is 1 or more sulfonic acids selected from the group essentially consisting of methanesulfonic acid, trifluoromethanesulfonic acid and p-toluenesulfonic acid.

4. The method according to any one of claims 1 to 3, wherein an organic amine compound is used as the basic compound.

5. The method according to claim 4, wherein at least one of triethylamine and diisopropylamine is used as the organic amine compound.

6. The method according to any one of claims 1 to 5, wherein Step A to Step C are repeated multiple times by obtaining the compound represented by the formula (VI) having ONBn as the Pro¹ in Step C and using the obtained compound represented by the formula (VI) as the compound represented by the formula (III) in Step B.

7. The method according to any one of claims 1 to 6, wherein Step A to Step C are repeated multiple times by obtaining the compound represented by the formula (VI) having NH₂ or ONBn as the Pro¹ in Step C and using the obtained compound represented by the formula (VI) as the compound represented by the formula (I) in Step A.

8. The method according to any one of claims 1 to 7, the method further comprising the step to deprotect the reactive side chain functional group of at least the compound represented by the formula (VI).

9. The method according to any one of claims 1 to 8, wherein a solvent is used in Step C and an amide solvent is used as the solvent.

10. The method according to claim 9, wherein the amide solvent is 1 or more amide solvents selected from the group essentially consisting of dimethylformamide, dimethylacetamide and dibutylformamide.

11. The method according to any one of claims 1 to 10, wherein the long-chain peptide is exenatide.

12. The method according to claim 8, wherein
peptide fragments having the following amino acid sequences are used:
F¹: Ala-Pro-Pro-Pro-Ser
F²: Gly-Pro-Ser-Ser-Gly
F³: Asn-Gly
F⁴: Phe-Ile-Glu-Trp-Leu-Lys
F⁵: Glu-Ala-Val-Arg-Leu
F⁶: Ser-Lys-Gln-Met-Glu-Glu
F⁷: Thr-Phe-Thr-Ser-Asp-Leu
F⁸: His-Gly-Glu-Gly,
and the peptide fragment F^{s} wherein an N-terminal site is protected with Boc, a C-terminal site is protected with Pro¹, and s is any one of integers of 1 or more and 7 or less is condensated with the peptide fragment F^{s+1} wherein an N-terminal site is protected with Boc and C-terminal site is protected with ONBn by using the peptide fragment F^{s} as the compound represented by the formula (I) and using the peptide fragment F^{s+1} as the compound represented by the formula (III).

13. The method according to claim 12, the method comprising
a step to produce a peptide fragment F⁹ having the following amino acid sequence by using the peptide fragment F¹ wherein an N-terminal site is protected with Boc and a C-terminal site is protected with NH₂ as the compound represented by the formula (I) and using the peptide fragment F² wherein an N-terminal site is protected with Boc and a C-terminal site is protected with ONBn as the compound represented by the formula (III),
F⁹: Gly-Pro-Ser-Ser-Gly-Ala-Pro-Pro-Pro-Ser
a step to produce a peptide fragment F¹⁰ having the following amino acid sequence by using the peptide fragment F³ wherein an N-terminal site is protected with Boc and a C-terminal site is protected with ONBn as the compound represented by the formula (I) and using the peptide fragment F⁴ wherein an N-terminal site is protected with Boc and a C-terminal site is protected with ONBn as the compound represented by the formula (III),
F¹⁰: Phe-Ile-Glu-Trp-Leu-Lys-Asn-Gly
a step to produce a peptide fragment F¹¹ having the following amino acid sequence by using the peptide fragment F⁷ wherein an N-terminal site is protected with Boc and a C-terminal site is protected with ONBn as the compound represented by the formula (I) and using the peptide fragment F⁸ wherein an N-terminal site is protected with Boc and a C-terminal site is protected with ONBn as the compound represented by the formula (III),
F¹¹: His-Gly-Glu-Gly-Thr-Phe-Thr-Ser-Asp-Leu
a step to produce a peptide fragment F¹² having the following amino acid sequence by using the peptide fragment F¹⁰ wherein an N-terminal site is protected with Boc and a C-terminal site is protected with ONBn as the compound represented by the formula (I) and using the peptide fragment F⁵ wherein an N-terminal site is protected with Boc and a C-terminal site is protected with ONBn as the compound represented by the formula (III),
F¹²: Glu-Ala-Val-Arg-Leu-Phe-Ile-Glu-Trp-Leu-Lys-Asn-Gly
a step to produce a peptide fragment F¹³ having the following amino acid sequence by using the peptide fragment F⁹ wherein an N-terminal site is protected with Boc and a C-terminal site is protected with NH₂ as the compound represented by the formula (I) and using the peptide fragment F¹² wherein an N-terminal site is protected with Boc and a C-terminal site is protected with ONBn as the compound represented by the formula (III),
F¹³: Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
a step to produce a peptide fragment F¹⁴ having the following amino acid sequence by using the peptide fragment F¹³ wherein an N-terminal site is protected with Boc and a C-terminal site is protected with NH₂ as the compound represented by the formula (I) and using the peptide fragment F⁶ wherein an N-terminal site is protected with Boc and a C-terminal site is protected with ONBn as the compound represented by the formula (III),
F¹⁴: Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
a step to produce a peptide fragment F¹⁵ having the following amino acid sequence by using the peptide fragment F¹⁴ wherein an N-terminal site is protected with Boc and a C-terminal site is protected with NH₂ as the compound represented by the formula (I) and using the peptide fragment F¹¹ wherein an N-terminal site is protected with Boc and a C-terminal site is protected with ONBn as the compound represented by the formula (III),
F¹⁵: His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser
and a step to deprotect an N-terminal site and a reactive side chain functional group of the peptide fragment F¹⁵.


SEQUENCE LISTING

<110> KANEKA CORPORATION

<120> METHOD FOR PRODUCING LONG-CHAIN PEPTIDE

<130> F18-110EP01P

<140> JPPCT/JP2019/000151
<141> 2018-03-29

<160> 36

<170> BiSSAP 1.3.6

<210> 1
<211> 5
<212> PRT
<213> Artificial Sequence

<220>
<223> peptide fragment F1

<400> 1
Ala Pro Pro Pro Ser
1               5

<210> 2
<211> 5
<212> PRT
<213> Artificial Sequence

<220>
<223> peptide fragment F2

<400> 2
Gly Pro Ser Ser Gly
1               5

<210> 3
<211> 6
<212> PRT
<213> Artificial Sequence

<220>
<223> peptide fragment F4

<400> 3
Phe Ile Glu Trp Leu Lys
1               5

<210> 4
<211> 5
<212> PRT
<213> Artificial Sequence

<220>
<223> peptide fragment F5

<400> 4
Glu Ala Val Arg Leu
1               5

<210> 5
<211> 6
<212> PRT
<213> Artificial Sequence

<220>
<223> peptide fragment F6

<400> 5
Ser Lys Gln Met Glu Glu
1               5

<210> 6
<211> 6
<212> PRT
<213> Artificial Sequence

<220>
<223> peptide fragment F7

<400> 6
Thr Phe Thr Ser Asp Leu
1               5

<210> 7
<211> 4
<212> PRT
<213> Artificial Sequence

<220>
<223> peptide fragment F8

<400> 7
His Gly Glu Gly
1

<210> 8
<211> 10
<212> PRT
<213> Artificial Sequence

<220>
<223> peptide fragment F9

<400> 8
Gly Pro Ser Ser Gly Ala Pro Pro Pro Ser
1               5                   10

<210> 9
<211> 8
<212> PRT
<213> Artificial Sequence

<220>

<223> peptide fragment F10

<400> 9
Phe Ile Glu Trp Leu Lys Asn Gly
1               5

<210> 10
<211> 10
<212> PRT
<213> Artificial Sequence


<220>
<223> peptide fragment F11

<400> 10
His Gly Glu Gly Thr Phe Thr Ser Asp Leu
1               5                   10

<210> 11
<211> 13
<212> PRT
<213> Artificial Sequence


<220>
<223> peptide fragment F12

<400> 11
Glu Ala Val Arg Leu Phe Ile Glu Trp Leu Lys Asn Gly
1               5                   10

<210> 12
<211> 23
<212> PRT
<213> Artificial Sequence


<220>
<223> peptide fragment F13

<400> 12
Glu Ala Val Arg Leu Phe Ile Glu Trp Leu Lys Asn Gly Gly Pro Ser
1               5                   10                  15
Ser Gly Ala Pro Pro Pro Ser
            20

<210> 13
<211> 29
<212> PRT
<213> Artificial Sequence


<220>
<223> peptide fragment F14

<400> 13
Ser Lys Gln Met Glu Glu Glu Ala Val Arg Leu Phe Ile Glu Trp Leu
1               5                   10                  15
Lys Asn Gly Gly Pro Ser Ser Gly Ala Pro Pro Pro Ser
            20                  25

<210> 14

<211> 39
<212> PRT
<213> Artificial Sequence

<220>
<223> peptide fragment F15

<400> 14
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210> 15
<211> 39
<212> PRT
<213> Artificial Sequence

<220>
<223> Exenatide

<220>
<221> MOD_RES
<222> 39
<223> AMIDATION

<400> 15
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210> 16
<211> 5
<212> PRT
<213> Artificial Sequence

<220>
<221> MOD_RES
<222> 1
<223> t-butoxycarbonyl (Boc)

<220>
<223> see fragment F2 (example 1)

<220>
<221> MOD_RES
<222> 3
<223> benzyl (Bzl)

<220>
<221> MOD_RES
<222> 4
<223> benzyl (Bzl)

<220>

<221> MOD_RES
<222> 5
<223> hydroxy (OH)

<400> 16
Gly Pro Ser Ser Gly
1               5

<210> 17
<211> 5
<212> PRT
<213> Artificial Sequence

<220>
<221> MOD_RES
<222> 1
<223> t-butoxycarbonyl (Boc)

<220>
<223> see fragment F2 (example 1)

<220>
<221> MOD_RES
<222> 3
<223> benzyl (Bzl)

<220>
<221> MOD_RES
<222> 4
<223> benzyl (Bzl)

<220>
<221> MOD_RES
<222> 5
<223> -O(4-NBn); nitrobenzyl (NBn)

<400> 17
Gly Pro Ser Ser Gly
1               5

<210> 18
<211> 10
<212> PRT
<213> Artificial Sequence

<220>
<221> MOD_RES
<222> 1
<223> t-butoxycarbonyl (Boc)

<220>
<223> see fragment F9 (example 1)

<220>
<221> MOD_RES
<222> 3
<223> benzyl (Bzl)

<220>
<221> MOD_RES
<222> 4

<223> benzyl (Bzl)

<220>
<221> MOD_RES
<222> 10
<223> benzyl (Bzl)

<220>
<221> MOD_RES
<222> 10
<223> amino group (-NH2)

<400> 18
Gly Pro Ser Ser Gly Ala Pro Pro Pro Ser
1               5                   10

<210> 19
<211> 5
<212> PRT
<213> Artificial Sequence

<220>
<221> MOD_RES
<222> 1
<223> t-butoxycarbonyl (Boc)

<220>
<223> see fragment F1 (example 1)

<220>
<221> MOD_RES
<222> 5
<223> benzyl (Bzl)

<220>
<221> MOD_RES
<222> 5
<223> hydroxy (OH)

<400> 19
Ala Pro Pro Pro Ser
1               5

<210> 20
<211> 6
<212> PRT
<213> Artificial Sequence

<220>
<221> MOD_RES
<222> 1
<223> t-butoxycarbonyl (Boc)

<220>
<223> see fragment F4 (example 2)

<220>
<221> MOD_RES
<222> 3
<223> benzyloxy (OBzl)

<220>
<221> MOD_RES
<222> 4
<223> aldehyde (CHO)

<220>
<221> MOD_RES
<222> 6
<223> 2-chlorobenzyloxycarbonyl (Cl-Z)

<220>
<221> MOD_RES
<222> 6
<223> hydroxy (OH)

<400> 20
Phe Ile Glu Trp Leu Lys
1               5

<210> 21
<211> 6
<212> PRT
<213> Artificial Sequence

<220>
<221> MOD_RES
<222> 1
<223> t-butoxycarbonyl (Boc)

<220>
<223> see fragment F4 (example 2)

<220>
<221> MOD_RES
<222> 3
<223> benzyloxy (OBzl)

<220>
<221> MOD_RES
<222> 4
<223> aldehyde (CHO)

<220>
<221> MOD_RES
<222> 6
<223> 2-chlorobenzyloxycarbonyl (Cl-Z)

<220>
<221> MOD_RES
<222> 6
<223> -O(4-NBn); nitrobenzyl (NBn)

<400> 21
Phe Ile Glu Trp Leu Lys
1               5

<210> 22
<211> 8
<212> PRT
<213> Artificial Sequence

<220>
<221> MOD_RES
<222> 1
<223> t-butoxycarbonyl (Boc)

<220>
<223> see fragment F10 (example 2)

<220>
<221> MOD_RES
<222> 3
<223> benzyloxy (OBzl)

<220>
<221> MOD_RES
<222> 4
<223> aldehyde (CHO)

<220>
<221> MOD_RES
<222> 6
<223> 2-chlorobenzyloxycarbonyl (Cl-Z)

<220>
<221> MOD_RES
<222> 8
<223> -O(4-NBn); nitrobenzyl (NBn)

<400> 22
Phe Ile Glu Trp Leu Lys Asn Gly
1               5

<210> 23
<211> 4
<212> PRT
<213> Artificial Sequence



<220>
<221> MOD_RES
<222> 1
<223> t-butoxycarbonyl (Boc)

<220>
<221> MOD_RES
<222> 1
<223> benzyloxymethyl (BOM)

<220>
<223> see fragment F8 (example 3)

<220>
<221> MOD_RES
<222> 3
<223> benzyloxy (OBzl)

<220>
<221> MOD_RES
<222> 4
<223> hydroxy (OH)

<400> 23
His Gly Glu Gly

1

<210> 24
<211> 4
<212> PRT
<213> Artificial Sequence

<220>
<221> MOD_RES
<222> 1
<223> t-butoxycarbonyl (Boc)

<220>
<221> MOD_RES
<222> 1
<223> benzyloxymethyl (BOM)

<220>
<223> see fragment F8 (example 3)

<220>
<221> MOD_RES
<222> 3
<223> benzyloxy (OBzl)

<220>
<221> MOD_RES
<222> 4
<223> -O(4-NBn); nitrobenzyl (NBn)

<400> 24
His Gly Glu Gly
1

<210> 25
<211> 10
<212> PRT
<213> Artificial Sequence

<220>
<221> MOD_RES
<222> 1
<223> t-butoxycarbonyl (Boc)

<220>
<221> MOD_RES
<222> 1
<223> benzyloxymethyl (BOM)

<220>
<223> see fragment F11 (example 3)

<220>
<221> MOD_RES
<222> 3
<223> benzyloxy (OBzl)

<220>
<221> MOD_RES
<222> 5
<223> benzyl (Bzl)

<220>
<221> MOD_RES
<222> 7
<223> benzyl (Bzl)

<220>
<221> MOD_RES
<222> 8
<223> benzyl (Bzl)

<220>
<221> MOD_RES
<222> 9
<223> benzyloxy (OBzl)

<220>
<221> MOD_RES
<222> 10
<223> -O(4-NBn); nitrobenzyl (NBn)

<400> 25
His Gly Glu Gly Thr Phe Thr Ser Asp Leu
1               5                   10

<210> 26
<211> 6
<212> PRT
<213> Artificial Sequence

<220>
<221> MOD_RES
<222> 1
<223> t-butoxycarbonyl (Boc)

<220>
<221> MOD_RES
<222> 1
<223> benzyl (Bzl)

<220>
<223> see fragment F7 (example 3)

<220>
<221> MOD_RES
<222> 3
<223> benzyl (Bzl)

<220>
<221> MOD_RES
<222> 4
<223> benzyl (Bzl)

<220>
<221> MOD_RES
<222> 5
<223> benzyloxy (OBzl)

<220>
<221> MOD_RES
<222> 6
<223> -O(4-NBn); nitrobenzyl (NBn)

<400> 26
Thr Phe Thr Ser Asp Leu
1               5

<210> 27
<211> 5
<212> PRT
<213> Artificial Sequence

<220>
<221> MOD_RES
<222> 1
<223> t-butoxycarbonyl (Boc)

<220>
<221> MOD_RES
<222> 1
<223> benzyloxy (OBzl)

<220>
<223> see fragment F5 (example 4)

<220>
<221> MOD_RES
<222> 4
<223> (benzyloxycarbonyl)2 (Z)2

<220>
<221> MOD_RES
<222> 5
<223> hydroxy (OH)

<400> 27
Glu Ala Val Arg Leu
1               5

<210> 28
<211> 5
<212> PRT
<213> Artificial Sequence

<220>
<221> MOD_RES
<222> 1
<223> t-butoxycarbonyl (Boc)

<220>
<221> MOD_RES
<222> 1
<223> benzyloxy (OBzl)

<220>
<223> see fragment F5 (example 4)

<220>
<221> MOD_RES
<222> 4
<223> (benzyloxycarbonyl)2 (Z)2

<220>

<221> MOD_RES
<222> 5
<223> -O(4-NBn); nitrobenzyl (NBn)

<400> 28
Glu Ala Val Arg Leu
1               5

<210> 29
<211> 13
<212> PRT
<213> Artificial Sequence

<220>
<221> MOD_RES
<222> 1
<223> t-butoxycarbonyl (Boc)

<220>
<221> MOD_RES
<222> 1
<223> benzyloxy (OBzl)

<220>
<223> see fragment F12 (example 4)

<220>
<221> MOD_RES
<222> 4
<223> (benzyloxycarbonyl)2 (Z)2

<220>
<221> MOD_RES
<222> 8
<223> benzyloxy (OBzl)

<220>
<221> MOD_RES
<222> 9
<223> aldehyde (CHO)

<220>
<221> MOD_RES
<222> 11
<223> 2-chlorobenzyloxycarbonyl (Cl-Z)

<220>
<221> MOD_RES
<222> 13
<223> -O(4-NBn); nitrobenzyl (NBn)

<400> 29
Glu Ala Val Arg Leu Phe Ile Glu Trp Leu Lys Asn Gly
1               5                   10

<210> 30
<211> 13
<212> PRT
<213> Artificial Sequence

<220>

<221> MOD_RES
<222> 1
<223> t-butoxycarbonyl (Boc)

<220>
<221> MOD_RES
<222> 1
<223> benzyloxy (OBzl)

<220>
<223> see fragment F12 (example 5)

<220>
<221> MOD_RES
<222> 4
<223> (benzyloxycarbonyl)2 (Z)2

<220>
<221> MOD_RES
<222> 8
<223> benzyloxy (OBzl)

<220>
<221> MOD_RES
<222> 9
<223> aldehyde (CHO)

<220>
<221> MOD_RES
<222> 11
<223> 2-chlorobenzyloxycarbonyl (Cl-Z)

<220>
<221> MOD_RES
<222> 13
<223> hydroxy (OH)

<400> 30
Glu Ala Val Arg Leu Phe Ile Glu Trp Leu Lys Asn Gly
1               5                   10

<210> 31
<211> 23
<212> PRT
<213> Artificial Sequence

<220>
<221> MOD_RES
<222> 1
<223> t-butoxycarbonyl (Boc)

<220>
<221> MOD_RES
<222> 1
<223> benzyloxy (OBzl)

<220>
<223> see fragment F13 (example 5)

<220>
<221> MOD_RES

<222> 4
<223> (benzyloxycarbonyl)2 (Z)2

<220>
<221> MOD_RES
<222> 8
<223> benzyloxy (OBzl)

<220>
<221> MOD_RES
<222> 9
<223> aldehyde (CHO)

<220>
<221> MOD_RES
<222> 11
<223> 2-chlorobenzyloxycarbonyl (Cl-Z)

<220>
<221> MOD_RES
<222> 16
<223> benzyl (Bzl)

<220>
<221> MOD_RES
<222> 17
<223> benzyl (Bzl)

<220>
<221> MOD_RES
<222> 23
<223> benzyl (Bzl)

<220>
<221> MOD_RES
<222> 23
<223> amino (NH2)

<400> 31
Glu Ala Val Arg Leu Phe Ile Glu Trp Leu Lys Asn Gly Gly Pro Ser
1               5                   10                  15
Ser Gly Ala Pro Pro Pro Ser
            20

<210> 32
<211> 6
<212> PRT
<213> Artificial Sequence


<220>
<221> MOD_RES
<222> 1
<223> t-butoxycarbonyl (Boc)

<220>
<221> MOD_RES
<222> 1
<223> benzyl (Bzl)

<220>
<223> see fragment F6 (example 6)

<220>
<221> MOD_RES
<222> 2
<223> 2-chlorobenzyloxycarbonyl (Cl-Z)

<220>
<221> MOD_RES
<222> 4
<223> (O)

<220>
<221> MOD_RES
<222> 5
<223> benzyloxy (OBzl)

<220>
<221> MOD_RES
<222> 6
<223> benzyloxy (OBzl)

<220>
<221> MOD_RES
<222> 6
<223> hydroxy (OH)

<400> 32
Ser Lys Gln Met Glu Glu
1               5

<210> 33
<211> 6
<212> PRT
<213> Artificial Sequence

<220>
<221> MOD_RES
<222> 1
<223> t-butoxycarbonyl (Boc)

<220>
<221> MOD_RES
<222> 1
<223> benzyl (Bzl)

<220>
<223> see fragment F6 (example 6)

<220>
<221> MOD_RES
<222> 2
<223> 2-chlorobenzyloxycarbonyl (Cl-Z)

<220>
<221> MOD_RES
<222> 4
<223> (O)

<220>
<221> MOD_RES
<222> 5
<223> benzyloxy (OBzl)

<220>
<221> MOD_RES
<222> 6
<223> benzyloxy (OBzl)

<220>
<221> MOD_RES
<222> 6
<223> -O(4-NBn); nitrobenzyl (NBn)

<400> 33
Ser Lys Gln Met Glu Glu
1               5

<210> 34
<211> 29
<212> PRT
<213> Artificial Sequence

<220>
<221> MOD_RES
<222> 1
<223> t-butoxycarbonyl (Boc)

<220>
<221> MOD_RES
<222> 1
<223> benzyl (Bzl)

<220>
<223> see fragment F14 (example 6)

<220>
<221> MOD_RES
<222> 2
<223> 2-chlorobenzyloxycarbonyl (Cl-Z)

<220>
<221> MOD_RES
<222> 4
<223> (O)

<220>
<221> MOD_RES
<222> 5
<223> benzyloxy (OBzl)

<220>
<221> MOD_RES
<222> 6
<223> benzyloxy (OBzl)

<220>
<221> MOD_RES
<222> 7
<223> benzyloxy (OBzl)

<220>
<221> MOD_RES
<222> 10
<223> (benzyloxycarbonyl)2 (Z)2

<220>
<221> MOD_RES
<222> 14
<223> benzyloxy (OBzl)

<220>
<221> MOD_RES
<222> 15
<223> aldehyde (CHO)

<220>
<221> MOD_RES
<222> 17
<223> 2-chlorobenzyloxycarbonyl (Cl-Z)

<220>
<221> MOD_RES
<222> 22
<223> benzyl (Bzl)

<220>
<221> MOD_RES
<222> 23
<223> benzyl (Bzl)

<220>
<221> MOD_RES
<222> 29
<223> benzyl (Bzl)

<220>
<221> MOD_RES
<222> 29
<223> amino (NH2)

<400> 34
Ser Lys Gln Met Glu Glu Glu Ala Val Arg Leu Phe Ile Glu Trp Leu
1               5                   10                  15
Lys Asn Gly Gly Pro Ser Ser Gly Ala Pro Pro Pro Ser
            20                  25

<210> 35
<211> 10
<212> PRT
<213> Artificial Sequence

<220>
<221> MOD_RES
<222> 1
<223> t-butoxycarbonyl (Boc)

<220>
<221> MOD_RES
<222> 1
<223> benzyloxymethyl (BOM)

<220>
<223> see fragment F11 (example 7)

<220>
<221> MOD_RES
<222> 3

<223> benzyloxy (OBzl)

<220>
<221> MOD_RES
<222> 5
<223> benzyl (Bzl)

<220>
<221> MOD_RES
<222> 7
<223> benzyl (Bzl)

<220>
<221> MOD_RES
<222> 8
<223> benzyl (Bzl)

<220>
<221> MOD_RES
<222> 9
<223> benzyloxy (OBzl)

<220>
<221> MOD_RES
<222> 10
<223> hydroxy (OH)

<400> 35
His Gly Glu Gly Thr Phe Thr Ser Asp Leu
1               5                   10

<210> 36
<211> 39
<212> PRT
<213> Artificial Sequence

<220>
<221> MOD_RES
<222> 1
<223> t-butoxycarbonyl (Boc)

<220>
<221> MOD_RES
<222> 1
<223> benzyloxymethyl (BOM)

<220>
<223> see fragment F15 (example 7)

<220>
<221> MOD_RES
<222> 3
<223> benzyloxy (OBzl)

<220>
<221> MOD_RES
<222> 5
<223> benzyl (Bzl)

<220>
<221> MOD_RES
<222> 7

<223> benzyl (Bzl)

<220>
<221> MOD_RES
<222> 8
<223> benzyl (Bzl)

<220>
<221> MOD_RES
<222> 9
<223> benzyloxy (OBzl)

<220>
<221> MOD_RES
<222> 11
<223> benzyl (Bzl)

<220>
<221> MOD_RES
<222> 12
<223> 2-chlorobenzyloxycarbonyl (Cl-Z)

<220>
<221> MOD_RES
<222> 14
<223> (O)

<220>
<221> MOD_RES
<222> 15
<223> benzyloxy (OBzl)

<220>
<221> MOD_RES
<222> 16
<223> benzyloxy (OBzl)

<220>
<221> MOD_RES
<222> 17
<223> benzyloxy (OBzl)

<220>
<221> MOD_RES
<222> 20
<223> (benzyloxycarbonyl)2 (Z)2

<220>
<221> MOD_RES
<222> 24
<223> benzyloxy (OBzl)

<220>
<221> MOD_RES
<222> 25
<223> aldehyde (CHO)

<220>
<221> MOD_RES
<222> 27
<223> 2-chlorobenzyloxycarbonyl (Cl-Z)

<220>

<221> MOD_RES
<222> 32
<223> benzyl (Bzl)

<220>
<221> MOD_RES
<222> 33
<223> benzyl (Bzl)

<220>
<221> MOD_RES
<222> 39
<223> benzyl (Bzl)

<220>
<221> MOD_RES
<222> 39
<223> amino (NH2)

<400> 36
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35